# EUROPÄISCHE PATENTANMELDUNG

(11) **EP 4 124 332 A1**
(43) Veröffentlichungstag der Anmeldung: **01.02.2023**
(21) Anmeldenummer: 22174378.4
(22) Anmeldetag: 19.05.2022
(51) Int. Cl.: A61K 8/04, A61K 8/60, A61K 8/73, A61K 8/81, A61K 8/92, A61Q 5/06

(54) **HAARGEL ENTHALTEND EIN NATÜRLICHES STYLINGPOLYMER**

(30) Priorität: 23.06.2021 DE 102021206451
(71) Anmelder: Beiersdorf AG, 20245 Hamburg (DE)
(72) Erfinder: Budnick, Nina, Hamburg 22147 (DE); Nemnich, Julia, 22589 Hamburg (DE)
(74) Vertreter: Beiersdorf AG

(57) **Zusammenfassung**

Haargel, das bei dem weitgehend oder ganz auf den Einsatz synthetischer fixierender, Filmbildenden und/oder verdickender Verbindungen verzichtet wird, dabei eine gute Festigungsleistung und gute Halteeigenschaften der Frisur aufweist.

## Beschreibung

Die vorliegende Erfindung betrifft ein Haargel, das eine gute Festigungsleistung und gute Halteeigenschaften der Frisur aufweist. Darüber hinaus lässt sich dieses Haargel so konfektionieren, dass es aus einer Tube entnommen werden kann. Weiterhin ist das Haargel so ausgestaltet, dass weitgehend oder ganz auf synthetische fixierende, Film-bildende und/oder verdickende Verbindungen verzichtet werden kann.

Seit Ende des 19. Jahrhunderts wurden gezielt Produkte zur Haarpflege entwickelt. Dies führte zu einer Vielzahl von Präparaten, sowohl für die allgemeine Haarpflege als auch zur Behebung von Anomalien des Haares und der Kopfhaut. Im Allgemeinen werden heutzutage Haarpflegekosmetika verwendet, welche dazu bestimmt sind, nach dem Einwirken entweder aus dem Haar wieder ausgespült zu werden oder auf dem Haar zu verbleiben.

Der ganze menschliche Körper mit Ausnahme der Lippen, der Handinnenflächen und der Fußsohlen ist behaart, zum Großteil allerdings mit kaum sichtbaren Wollhärchen. Wegen der vielen Nervenenden an der Haarwurzel reagieren Haare empfindlich auf äußere Einflüsse wie Wind oder Berührung und sind daher ein nicht zu unterschätzender Bestandteil des Tastsinns. Die wichtigste Funktion des menschlichen Kopfhaares dürfte allerdings heute darin bestehen, das Aussehen des Menschen in charakteristischer Weise mitzugestalten. Ähnlich wie die Haut erfüllt das Haar eine soziale Funktion, da es über sein Erscheinungsbild erheblich zu zwischenmenschlichen Beziehungen und zum Selbstwertgefühl des Individuums beiträgt.

Das Haar besteht aus dem frei aus der Haut herausragenden Haarschaft - dem keratinisierten (toten) Teil, der das eigentlich sichtbare Haar darstellt - und der in der Haut steckenden Haarwurzel - dem lebenden Teil, in dem das sichtbare Haar ständig neu gebildet wird. Der Haarschaft ist aus drei Schichten aufgebaut: Der zentrale Teil wird als Haarmark oder Medulla bezeichnet. Dieser Teil ist allerdings beim Menschen zurückgebildet und fehlt oft. An den zentralen Teil schließt sich die Haarrinde oder Cortex an. Diese Faserschicht besteht aus verhornten Faserzellen und bestimmt die Festigkeit und Elastizität des Haares. In dieser Schicht sind auch die Farbpigmente enthalten. Außen um die Faserschicht ist die Schuppenschicht oder Cuticula angeordnet. Diese Schicht ist mehrlagig, sehr dünn und durchsichtig.

Das menschliche Haar ist, sofern keine krankhaften Veränderungen vorliegen, in seinem frisch nachgewachsenen Zustand praktisch nicht zu verbessern. Der in der Nähe der Kopfhaut befindliche Teil eines Haares weist dementsprechend eine nahezu geschlossene Schuppenschicht auf.

Haargele können der Gruppe der Haar-fixierenden Kosmetika zugerechnet werden. Die Verwendung dieser Gruppe von Kosmetika dient in erster Linie dazu, die Frisur des Haares zu fixieren und über längere Zeit zu erhalten. Natürliches, unbehandeltes Haar, auch in frisch gewaschenem Zustand, hängt oft gerade und mit wenig Fülle herab. Die Verbraucher haben jedoch vielfach den Wunsch, ihr Haar auf eine andere Weise zu gestalten, zu frisieren und zu stylen. Diese Frisur soll einfach zu formen sein und auch über längere Zeit erhalten bleiben.

Haar-fixierende Kosmetika können auf die nassen Haare aufgetragen werden und während des Trocknungsvorgangs werden die Haare in die gewünschte Form gebracht und fixiert. Dies sind beispielsweise Festigerlösungen, auch als Pumpfestiger oder Schaumfestiger angeboten. Haarsprays, sowohl in Form von Aerosolsprays oder Pumpsprays werden auf das trockene, bereits gestylte Haar aufgetragen und dienen dann der Fixierung der Haare.

Haargele können als verdickte Haarfestiger angesehen werden. Diese Produkte lassen sich auf das nasse oder auf das trockene Haar aufgetragen. Das Lösungsmittel des Haargels trocknet und auf dem Haar bleibt ein klarer, idealerweise flexibler Film zurück, der die Frisur fixiert.

Haargele sind an sich bekannt. Es gibt auch eine Vielzahl von Dokumenten des StdT in Bezug auf Haargele, von denen hier nur einige wenige genannt werden.

Im Dokument DE 10354015 A1 werden verdickte Haarbehandlungsmittel offenbart, die u.a. einen polymeren Gelbildner, wenigstens ein haarfestigendes Polymer und Wasserglas enthalten. In den Beispielrezepturen werden auch Haargele beschrieben.

Im Dokument DE 102005020704 A1 werden Haargele beschrieben, die nachschäumende Zubereitungen sind. Die genannten Zubereitungen enthalten u.a. ein oder mehrere Film-bildende Polymere, ein oder mehrere verdickende Substanzen, Emulgatoren und lipophile Substanzen.

Im Dokument EP 1719499 B1 werden Haargele beschrieben, die neben festigenden Eigenschaften auch eine gute Kämmbarkeit des Haares bewirken. Die genannten Haargele enthalten u.a. ein anionisches Acrylatharz mit bestimmten Eigenschaften und anionische und/oder nichtionische polymere Verdicker.

Im Dokument EP 1981469 B1 werden Haargele offenbart, deren Verwendung nicht zu sichtbaren Rückständen auf dem Haar oder auf der Kleidung führt. Die genannten Haargele enthalten ein anionisches verdickendes Polymer, ein oder mehrere Film-bildende Polymere, bevorzugt ein VP/VA-Copolymer und ein hochmolekulares Polyethylenglycol, wobei die Fadenabrisszeit der Zubereitung in einem bestimmten Bereich liegt.

Wie aus dieser kurzen Übersicht zu erkennen ist, enthalten Haargele einen Verdicker, meist einen Acrylat-basierten Verdicker und ein oder mehrere Film-bildende Polymere, alles Verbindungen, die in der Regel synthetischer Natur sind. Es handelt sich beispielsweise um Carbomere, VP/VA-Polymere, Vinyl Caprolactam/VP/Dimethylaminoethylmathacrylate Copolymere, VA/Crotonate Copolymere, PVP, Acrylates/Ceteth-20 Itaconate Copolymere, Acrylates/Steareth-20 Itaconate Copolymere, Acrylates/Beheneth-25 Methacrylate Copolymere, Acrylates/Steareth-20 Methacrylate Copolymere, Polyquaternium-46 (Terpolymer aus vinylcaprolactam, vinylpyrrolidone und quaterniziertem vinylimidazole), Acrylate Copolymere, Acrylates/Hydroxyester Acrylates Copolymere, Acrylates/C10-30 Alkyl Acrylate Crosspolymere, Polyquaternium-16 (Copolymer aus Vinylpyrrolidone und quaternisiertem Vinylimidazol), Polyquaternium-11 (Copolymer aus Vinylpyrrolidone und quaternisiertem Dimethylaminoethyl Methacrylat), Polyquaternium-4 (quaternäres Ammoniumsalz von Hydroxyethylcellulose quaterniert mit Diallyl dimethyl ammonium Chloride); darüber hinaus werden in Haargelzubereitungen des Standes der Technik noch eine Vielzahl weiterer synthetischer Polymere eingesetzt.

Synthetische Polymere sind, wie der Name aussagt, synthetische Verbindungen, für die es in der Regel keine natürlichen Abbaumechanismen gibt. Die Folge ist, dass derartige Verbindungen nicht gut, sehr schlecht oder fast gar nicht biologisch abbaubar sind.

Wünschenswert ist jedoch der Einsatz von Polymeren in Haargelzubereitungen und allgemein in kosmetischen Zubereitungen, die natürliche Polymere sind oder Polymere natürlichen Ursprungs sind und somit in der Regel eine (gute) biologische Abbaubarkeit aufweisen.

Der Begriff "natürlich" im Zusammenhang mit natürlichen Inhaltsstoffen für Kosmetika ist in der ISO 16128 festgelegt. Gemäß dieser Richtlinie kann man natürliche, kosmetische Inhaltsstoffe zusammenfassend als Stoffe beschreiben, die von Pflanzen, Tieren, Mineralien oder Mikroorganismen erhältlich sind, eingeschlossen sind auch solche Stoffe, die von den genannten Quellen durch physikalische Prozesse, Fermentationsprozesse (nur solche Fermentationsprozesse, die auch in der Natur ablaufen und die zu Produkten führen, die auch in der Natur entstehen) und andere Herstellungsmethoden ohne beabsichtigte chemische Modifikation erhalten werden. Die Mikroorganismen dürfen nur solche sein, die nicht gentechnisch modifiziert wurden.

Im Sinne der vorliegenden Erfindung sind Substanzen natürlichen Ursprungs, insbesondere Polymere natürlichen Ursprungs, Verbindungen, die von natürlichen Substanzen/Polymeren durch Modifizierungen abgeleitet sind. Damit sind auch chemische Modifizierungen umfasst. Der natürliche Grundcharakter der Substanzen/Polymere bleibt jedoch erhalten.

Der Begriff der biologischen Abbaubarkeit beschreibt den Prozess des Abbaus von organischen Verbindungen durch Lebewesen, insbesondere Saprobionten. Im Idealfall entstehen anorganische Stoffe, wie beispielsweise CO₂, O₂ und Ammoniak; Verbindungen, die von Pflanzen und Mikroorganismen wieder für den Aufbau von organischen Verbindungen genutzt werden.

Organische, chemische Verbindungen, die leicht biologisch abbaubar sind, sind nach OECD 301, für kosmetische Zusammensetzungen meist nach OECD 301 B klassifiziert. Diese Substanzen oder Zusammensetzungen können rasch und vollständig abgebaut werden.

Organische, chemische Verbindungen, die nach OECD 302 klassifiziert sind, sind zwar eingeschränkt, jedoch grundsätzlich biologisch abbaubar.

In dem Dokument mit der Anmeldenummer DE102020200497.8 wird ein Haargel beschrieben, das eine festigende Wirkung erzielt und dabei natürliche oder Natur-basierte festigende Komponenten umfasst.

Die Aufgabe der vorliegenden Erfindung war es, ein Haargel zur Verfügung zu stellen, das Polymere, die natürliche Polymere sind oder Polymere natürlichen Ursprungs sind, enthält. Die Menge an synthetischen Polymeren, die einen Beitrag zu den festigenden Eigenschaften des Haargels leisten, sollte verringert werden oder es sollte komplett auf derartige Polymere verzichtet werden. Die erfindungsgemäßen Haargele sollten jedoch in den Anwendungseigenschaften, Festigung und Frisurerhalt, mit herkömmlichen Haargelen vergleichbar sein. Zusätzlich sollte das Haargel so ausgestaltet sein, dass eine Bereitstellung in einer Tube und die Entnahme daraus möglich ist.

Gelöst wurden die vorstehenden Aufgaben durch ein Haargel, das wenigstens eine natürliche, wasserlösliche, lineare Struktur-gebende Verbindung, insbesondere Pullulan enthält.

Im StdT sind bereits Dokumente veröffentlicht, die die Verwendung von Pullulan, offenbaren.

Das Dokument FR 3064474 B1 beschreibt die Verwendung einer Kombination von Pullulan oder Derivaten des Pullulans zusammen mit Alginsäure und/oder Hyaluronsäure zum Schutz oder zur Reparatur von Hautanhangsgebilden.

Das Dokument US 2020/0000701 A1 beschreibt Haar-Stylingzusammensetzungen, die Pullulan und wenigstens eine Cellulose-Ether-Verbindung enthalten.

Die Aufgabe der vorliegenden Erfindung war es also, ein Haargel zur Verfügung zu stellen, das wenigstens ein Polymer enthält, das ein natürliches Polymer oder ein Polymer natürlichen Ursprungs ist. Die Menge an synthetischen Polymeren, die einen Beitrag zu den festigenden Eigenschaften des Haargels leisten, sollte verringert werden oder es sollte komplett auf derartige Polymere verzichtet werden. Die erfindungsgemäßen Haargele sollten jedoch in den Anwendungseigenschaften, Festigung und Frisurerhalt, mit herkömmlichen Haargelen vergleichbar sein.

Gelöst werden die vorstehenden Aufgaben durch ein Haargel enthaltend
- Pullulan in einer Menge von 0,1 bis 15,0 Gew.-%,
- wenigstens einen natürlichen Verdicker und/oder wenigstens einen Verdicker natürlichen Ursprungs, mit der Maßgabe, dass der natürliche Verdicker nicht Pullulan ist,
- optional wenigstens ein weiteres Film- bildendes und/oder festigendes Polymer, wobei das Polymer ein natürliches Polymer oder ein Polymer natürlichen Ursprungs ist, mit der Maßgabe, dass das wenigstens eine weitere Film- bildende und/oder festigende Polymer nicht Pullulan, nicht der wenigstens eine natürliche Verdicker und/oder nicht der wenigstens eine Verdicker natürlichen Ursprungs ist und
- optional wenigstens ein nichtionisches Tensid und/oder PEG-40 gehärtetes Rizinusöl.

Das erfindungsgemäße Haargel ist eine kosmetische Zusammensetzung und bevorzugt zum Stylen des menschlichen Kopfhaares vorgesehen.

Das erfindungsgemäße Haargel ist eine wässrige Zusammensetzung; der Wassergehalt beträgt vorteilhaft von 70 bis 98 Gew.-%, bevorzugt von 75 bis 95 Gew.-%, bezogen auf das Gesamtgewicht des Haargels.

Das erfindungsgemäße Haargel enthält eine Struktur-gebende Verbindung. Diese Verbindung trägt zur Fixierung der Haare und zur Frisurgestaltung bei. Darüber hinaus unterstützt sie im Haargel auch den Halt der Frisur.

Die Struktur-gebende Verbindungen ist Pullulan. Pullulan ist ein α-1,6 verknüpftes Polysaccharid aus Maltotriose-Einheiten, das aus dem Pilz *Aureobasidium pullulans* gewonnen werden kann. Die Glucose-Einheiten der Maltotriose sind α-1,4-glycosidisch miteinander verknüpft. Pullulan ist gut in Wasser löslich.

Pullulan kann beispielsweise unter dem Handelsnamen Pullulan von der Firma Hayashibara Co., LTD. bezogen werden.

In dem erfindungsgemäßen Haargel ist Pullulan mit einem Gehalt von 0,3 bis 10,0 Gew.-%, bevorzugt von 0,5 bis 7,0 Gew.-% enthalten, bezogen auf das Gesamtgewicht des Haargels.

Das erfindungsgemäße Haargel enthält wenigstens einen natürlichen Verdicker und/oder wenigstens einen Verdicker natürlichen Ursprungs, mit der Maßgabe, dass der natürliche Verdicker nicht Pullulan ist. Verdicker können auch als "Hydrokolloide" bezeichnet werden, eine technologische Kurzbezeichnung für die an sich richtigere Bezeichnung "hydrophile Kolloide". Hydrokolloide sind Makromoleküle, die eine lineare Gestalt haben können und über intermolekulare Wechselwirkungskräfte verfügen, die Neben- und Hauptvalenzbindungen zwischen den einzelnen Molekülen und damit die Ausbildung eines netzartigen Gebildes ermöglichen. Sie sind teilweise wasserlösliche natürliche oder synthetische Polymere, die in wässrigen Systemen Gele oder viskose Lösungen bilden. Sie erhöhen die Viskosität des Wassers, indem sie entweder Wassermoleküle binden (Hydratation) oder aber das Wasser in ihre unter sich verflochtenen Makromoleküle aufnehmen und einhüllen, wobei sie gleichzeitig die Beweglichkeit des Wassers einschränken. Solche wasserlöslichen Polymere stellen eine große Gruppe chemisch sehr unterschiedlicher natürlicher und synthetischer Polymere dar, deren gemeinsames Merkmal ihre Löslichkeit oder Quellbarkeit in Wasser bzw. wässrigen Medien ist. Voraussetzung dafür ist, dass diese Polymere über eine für die Wasserlöslichkeit oder Wasserquellbarkeit ausreichende Anzahl an hydrophilen Gruppen besitzen und nicht zu stark vernetzt sind.

Von bekannten, in der Kosmetik und auch Dermatologie einsetzbaren, Hydrokolloiden werden im Zusammenhang mit dieser Erfindung vorteilhaft folgende Polymere eingesetzt, nämlich:
- organische, natürliche Verbindungen, wie beispielsweise Agar-Agar, Carrageen, Tragant, Gummi arabicum, Alginate/Alginsäure, Pektine, Polyosen, Guar-Mehl, Johannisbrotbaumkernmehl, Dextrine, Gelatine, Casein, Xanthan Gum, Gellan Gum, Succinoglycan Gum, Skleroglucan;
- organische, abgewandelte (modifizierte) Naturstoffe, wie z. B. Carboxymethylcellulose und andere Celluloseether, Hydroxyethyl- und -propylcellulose und dergleichen.

Bevorzugte verdickend wirkende Polymere sind Biopolymere aus verschiedenen Quellen; erfindungsgemäß vorteilhaft kann der wenigstens eine natürliche Verdicker aus der Gruppe Xanthan Gum, Carrageenan und/oder Alginsäure und/oder deren Salzen ausgewählt werden.

Xanthan Gum wird mit Hilfe von Mikroorganismen der Gattung Xanthomonas gewonnen. Xanthan Gum ist ein Polymer, das aus einer Hauptkette aus ß-1,4 glykosidisch verknüpften Glucosemolekülen besteht. An jedem zweiten Glucoserest hängt eine Seitenkette aus zwei Mannoseresten, einem Glucoronsäurerest und Pyruvat.

Xanthan Gum löst sich gut in heißem und kaltem Wasser, dabei bilden sich Einfachhelices und Doppelhelices. Es entsteht ein dreidimensionales Netzwerk.

Xanthan Gum kann beispielsweise unter der Handelsbezeichnung Keltrol CG-F V von der Firma CP Kelco bezogen werden.

Carrageenan, auch Carrageen, ist eine Sammelbezeichnung für eine Gruppe langkettiger Kohlenhydrate, die aus Rotalgenzellen gewonnen werden können. Bei Carrageen handelt es sich um lineare, anionische Hydrokolloide, die sich aufgrund ihrer chemischen Struktur unterscheiden lassen und unterschiedliche Eigenschaften aufweisen.

Diese verschiedenen Typen unterscheiden sich durch den Anteil an Galactose und 3,6-Anhydrogalactose sowie über die Anzahl an Sulfatgruppen. Die Sulfatgruppen können in der protonierten Form als freie Säuregruppen vorkommen, oder sie kommen als Salze vor. Dann ist der Verdicker ein Carrageenat. Carrageen ist kommerziell erhältlich.

Carrageenan kann beispielsweise unter der Handelsbezeichnung Gelcarin CP 379 NF von der Firma FMC Health and Nutrition bezogen werden.

Die Salze der Alginsäure (Algin) werden allgemein als Alginate bezeichnet. Alginat ist ein Polysaccharid, das aus den Uronsäuren α-L-Guluronsäure (GuIUA) und β-D-Mannuronsäure (ManUA) besteht. Die Uronsäuren sind 1,4-glycosidisch in wechselndem Verhältnis zu linearen Ketten miteinander verbunden. Es bilden sich homopolymere Bereiche, in denen Mannuronsäure oder Guluronsäure als Blöcke vorliegen. Diese Blöcke werden als GG- oder MM-Blöcke bezeichnet. Im Bereich der GG- und MM-Blöcke kommt es zu einer Art Faltstruktur, die bei der Gelierung eine wesentliche Rolle spielt. Insbesondere die GG-Blöcke bilden eine regelmäßige Zickzack-Struktur aus.

Algin kann beispielsweise unter der Handelsbezeichnung Hydagen 558P von der Firma BASF bezogen werden.

In dem erfindungsgemäßen Haargel liegt der wenigstens eine natürliche Verdicker und/oder der wenigstens eine Verdicker natürlichen Ursprungs vorteilhaft mit einem Gesamtgehalt von 0,1 bis 8,0 Gew.-%, bevorzugt 1,0 bis 6,0 Gew.-% vor, bezogen auf das Gesamtgewicht des Haargels.

Wenngleich nicht bevorzugt, so ist in dem Haargel in bestimmten Ausführungsformen zusätzlich zu dem wenigstens einen natürlichen Verdicker und/oder dem wenigstens einen Verdicker natürlichen Ursprungs wenigstens ein Verdicker synthetischen Ursprungs enthalten. Diese Verdicker synthetischen Ursprungs können ausgewählt werden aus der Gruppe Polyacryl- und Polymethacryl-Verbindungen, Vinylpolymere, Polycarbonsäuren, Polyether, Polyimine, Polyamide; bevorzugt aus der Gruppe Polyacryl-Verbindungen und Vinylpolymere und insbesondere bevorzugt Acrylates/Steareth-20 Methacrylate Crosspolymer.

In anderen, auch nicht bevorzugten Ausführungsformen, ist in dem Haargel statt des natürlichen Verdickers und/oder des Verdickers natürlichen Ursprungs nur wenigstens ein Verdicker synthetischen Ursprungs enthalten.

Wenn in dem Haargel wenigstens ein Verdicker synthetischen Ursprungs enthalten ist, so liegt er mit einem Gesamtgehalt von 0,1 bis 10,0 Gew.-%, bevorzugt 1,0 bis 8,0 Gew.-% vor, bezogen auf den Aktivgehalt und bezogen auf das Gesamtgewicht des Haargels.

Das erfindungsgemäße Haargel enthält optional wenigstens ein weiteres Film- bildende und/oder festigendes Polymer, wobei das Polymer ein natürliches Polymer oder ein Polymer natürlichen Ursprungs ist, mit der Maßgabe, dass das wenigstens eine weitere Film- bildende und/oder festigende Polymer nicht Pullulan, nicht der wenigstens eine natürliche Verdicker und/oder nicht der wenigstens eine Verdicker natürlichen Ursprungs ist. Diese Polymere bilden nach dem Trocknen des Lösungsmittels einen Film auf dem Haar oder den Haarsträhnen aus, wobei kleine Brücken zwischen den Haaren oder Haarsträhnen ausgebildet werden. Auf diese Weise gelingt eine Fixierung der jeweiligen Frisur über einen längeren Zeitraum. Festigende Polymere werden auch als Film-bildende Polymere bezeichnet, weil sie, wie beschrieben, einen Film auf dem Haar oder der Haarsträhne ausbilden können. Die Begriffe festigende Polymere und Film-bildenden Polymere werden oft fließend verwendet und überlappen in weiten Teilen. Im Rahmen der vorliegenden Anmeldung werden die Begriffe Film-bildendes Polymer, Filmbildner und festigendes Polymer synonym verwendet.

Ein vorteilhaftes Film-bildendes Polymer ist Natrium Polyitaconat (INCI-Bezeichnung: Sodium Polyitaconate). Es ist ein Homopolymer aus Itaconsäureresten. Dieses Polymer gilt als 100 % natürliches Polymer.

Natrium Polyitaconat kann beispielsweise von der Firma Nouryon unter dem Handelsnamen Amaze SP bezogen werden.

In dem erfindungsgemäßen Haargel ist Natrium Polyitaconat mit einem Gehalt von 0,5 bis 15,0 Gew.-%, bevorzugt von 1,0 bis 8,0 Gew.-% enthalten, bezogen auf das Gesamtgewicht des Haargels.

Das Polymer Acrylates/Hydroxyesters Acrylates Copolymer ist ein synthetisches Polymer, das aus den Monomeren Acrylsäure, Methacrylsäure, einfachen Estern dieser Säuren und Hydroxyacrylsäureestern gebildet wird. Dieses Polymer hat Film-bildende und festigende Eigenschaften. Es findet beispielsweise Anwendung in entsprechenden Haar-kosmetischen Zubereitungen. Im Rahmen der vorliegenden Erfindung wurde dieses Polymer in eine Haargel-Zubereitung eingearbeitet, die als Vergleichszubereitung dient.

Dieses Polymer gilt als sehr langsam biologisch abbaubar.

Acrylates/Hydroxyesters Acrylates Copolymer kann beispielsweise von der Firma Dow unter dem Handelsnamen Acudyne 180 bezogen werden.

In dem Haargel ist Acrylates/Hydroxyesters Acrylates Copolymer mit einem Gehalt von 0,5 bis 10,0 Gew.-%, bevorzugt von 0,8 bis 8,0 Gew.-% enthalten, bezogen auf das Gesamtgewicht des Haargels.

Das Polymer Acrylates/Steareth-20 Methacrylate Crosspolymer ist ebenfalls ein synthetisches Polymer, das aus den Monomeren Acrylsäure, Methacrylsäure, einfachen Estern dieser Säuren und Estern aus Methacrylsäure und Steareth-20 gebildet wird, die mit einem Allylether des Pentaerythritols oder einem Allylether des Trimethylolpropans vernetzt sind. Ein derartiges Polymer ist beispielsweise unter der Handelsbezeichnung Aculyn 88 von der Firma Dow erhältlich. Es wird als Film-bildend, aber auch als verdickend beschrieben.

Die biologische Abbaubarkeit wird als sehr langsam beschrieben.

Wenn in dem Haargel Acrylates/Steareth-20 Methacrylate Crosspolymer enthalten ist, so liegt das Acrylates/Steareth-20 Methacrylate Crosspolymer mit einem Gehalt von 0,1 bis 10,0 Gew.-%, bevorzugt von 1,0 bis 8,0 Gew.-% vor, bezogen auf das Gesamtgewicht des Haargels.

Im Rahmen der vorliegenden Erfindung wurde Pullulan in Kombination mit Acrylates/Steareth-20 Methacrylate Crosspolymer in eine Haargel-Zubereitung eingearbeitet und in Hinblick auf die Halteeigenschaften untersucht. In dieser Haargel-Zubereitung ist die Kombination von Pullulan, als natürlicher Struktur-gebender Verbindung und einem synthetischen Film-bildenden und verdickenden Polymer verwirklicht.

Im Rahmen der vorliegenden Erfindung ist es vorteilhaft, wenn das Haargel keine Cellulose-Ether-Verbindungen enthält. Cellulose-Ether lassen sich durch die folgende allgemeine Formel darstellen: 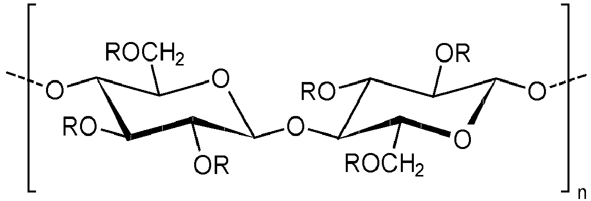 in der R ein Wasserstoff oder eine kurzkettige Alkylgruppe, beispielsweise Methylgruppe oder Ethylgruppe, darstellen kann.

Das erfindungsgemäße Haargel ist also frei von Cellulose-Ethern. Im Sinne der vorliegenden Erfindung bedeutet frei von, dass weniger als 0,01 Gew. %, bevorzugt weniger als 0,001 Gew.-%, besonders bevorzugt 0 Gew.-% der jeweiligen Substanz(en), bezogen auf das Gesamtgewicht der Zubereitung, vorliegen.

Im Rahmen bestimmter Ausführungsformen der vorliegenden Erfindung ist es vorteilhaft, wenn das Haargel frei ist von Acrylat-basierten Polymeren. Acrylat-basierte Polymere im Sinne der vorliegenden Erfindung sind Polymere, die als Monomerbausteine (Meth)Acrylsäure und Derivate dieser Säuren enthalten. Derivate sind strukturelle Analoga in Form von Estern, Ethern, Amiden, Hydroxylverbindungen und/oder Salzen. Bestimmte Ausführungsformen des erfindungsgemäßen Haargels enthalten also keine Acrylat-basierte Polymere.

Dennoch kann in einer möglichen Ausführungsform, wenn nicht komplett auf den Einsatz von synthetischen Polymeren verzichtet werden soll, wenigstens ein Acrylat-basiertes Polymer enthalten sein. Es wird dann ein Haargel bereitgestellt, das
- Pullulan in einer Menge von 0,1 bis 15,0 Gew.-%, bezogen auf das Gesamtgewicht des Haargels,
- wenigstens ein synthetisches Film- bildendes und verdickendes Polymer, ausgewählt aus Acrylat-basierten Polymeren, insbesondere Acrylates/Steareth-20 Methacrylate Crosspolymer und
- wenigstens ein nichtionisches Tensid und/oder PEG-40 gehärtetes Rizinusöl umfasst. In einer vorteilhaften Ausführungsform wird ein Haargel bereitgestellt, das
- Pullulan in einer Menge von 0,1 bis 15,0 Gew.-%, bezogen auf das Gesamtgewicht des Haargels,
- wenigstens einen natürlichen Verdicker und/oder wenigstens einen Verdicker natürlichen Ursprungs, ausgewählt aus Alginsäure und/oder ein oder mehrere Salze der Alginsäure und
- wenigstens ein nichtionisches Tensid und/oder PEG-40 gehärtetes Rizinusöl, insbesondere ein nichtionisches Tensid,
umfasst.

In einer weiteren vorteilhaften Ausführungsform wird ein Haargel bereitgestellt, das
- Pullulan in einer Menge von 0,1 bis 15,0 Gew.-%, bezogen auf das Gesamtgewicht des Haargels,
- wenigstens einen natürlichen Verdicker und/oder wenigstens einen Verdicker natürlichen Ursprungs, ausgewählt aus der Kombination von Xanthan Gum und Carrageenan und
- wenigstens ein weiteres Film- bildendes und/oder festigendes Polymer, wobei das Polymer ein natürliches Polymer oder ein Polymer natürlichen Ursprungs ist, mit der Maßgabe, dass das wenigstens eine weitere Film- bildende und/oder festigende Polymer nicht Pullulan, nicht der wenigstens eine natürliche Verdicker und/oder nicht der wenigstens eine Verdicker natürlichen Ursprungs ist, insbesondere Natrium Polyitaconat,
umfasst.

In einer weiteren, besonders vorteilhaften Ausführungsform wird ein Haargel bereitgestellt, das
- Pullulan in einer Menge von 0,1 bis 15 Gew.-%, bezogen auf das Gesamtgewicht des Haargels,
- wenigstens einen natürlichen Verdicker und/oder wenigstens einen Verdicker natürlichen Ursprungs, ausgewählt aus der Kombination von Xanthan Gum und Carrageenan,
- wenigstens ein weiteres Film- bildendes und/oder festigendes Polymer, wobei das wenigstens eine weitere Film- bildende und/oder festigende Polymer Natrium Polyitaconate ist und
- wenigstens ein nichtionisches Tensid und/oder PEG-40 gehärtetes Rizinusöl, insbesondere ein nichtionisches Tensid,
umfasst.

In dem erfindungsgemäßen Haargel kann optional wenigstens ein nichtionisches Tensid enthalten sein. Nicht-ionische Tenside bilden in wässrigem Medium keine Ionen.

Es ist bevorzugt, wenn das wenigstens eine nichtionische Tensid ausgewählt wird aus Alkylpolyglucosiden, insbesondere aus der Gruppe Laurylglucosid, Decylglucosid, Cocoglucosid und/oder Caprylyl/Capryl Glucoside.

Es ist ebenso bevorzugt, wenn das nichtionische Tensid ausgewählt wird aus Fettsäure-Polyglycerylestern. Diese Ester weisen mindestens zwei Glycerinmoleküle auf, die miteinander verknüpft sind. Die freien OH-Gruppen sind mit Fettsäuren verestert; wobei eine partielle Veresterung vorliegt, wenn nicht alle OH-Gruppen mit Fettsäuren verestert sind.

Partielle Fettsäure-Polyglycerylester lassen sich beispielsweise durch folgende Formel beschreiben: 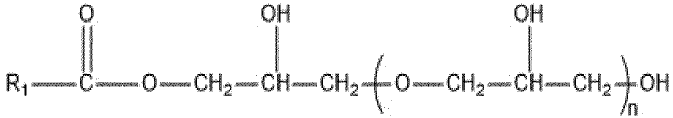 wobei R₁ ein linearer oder verzweigter, gesättigter oder ungesättigter Alkyl- bzw. Alkenylrest ist, wobei der Alkyl- bzw. Alkenylrest nur oder zum überwiegenden Teil Alkyl- bzw. Alkenylreste mit 6 bis 12 Kohlenstoffatomen, bevorzugt mit 6 bis 10 Kohlenstoffatomen aufweist und n eine ganze Zahl von 1 bis 11 ist.

Im Sinne der vorliegenden Erfindung bedeutet der Begriff überwiegend, dass mehr als 55 %, insbesondere mehr als 65 %, weiter insbesondere mehr als 80 % der genannten Reste 6 bis 12 Kohlenstoffatome, bevorzugt 6 bis 10 Kohlenstoffatome aufweisen.

Die Fettsäure-Polyglycerylester sind ungeladen, jedoch sind Sauerstoffatome der Glycerinmoleküle und Esterbindungen enthalten, die zur Hydrophilie beitragen, während die Alkyl/Alkylenreste der Fettsäuren zur Lipophilie beitragen. Es handelt sich bei den Fettsäure-Polyglycerylestern also um amphiphile Moleküle. Daher können die Fettsäure Polyglycerylester auch als nichtionische Tenside wirken und bezeichnet werden.

Beispiele für Fettsäure-Polyglycerylester sind Verbindungen wie Polyglyceryl-4 caprylate/caprate, Polyglyceryl-5 caprylate/caprate, Polyglyceryl-6 caprylate/caprate, Polyglyceryl-7 caprylate/caprate, Polyglyceryl-8 caprylate/caprate, Polyglyceryl-9 caprylate/caprate, Polyglyceryl-10 caprylate/caprate, Polyglyceryl-4 caprate, Polyglyceryl-5 caprate, Polyglyceryl-6 caprate, Polyglyceryl-7 caprate, Polyglyceryl-8 caprate, Polyglyceryl-9 caprate, Polyglyceryl-10 caprate, Polyglyceryl-4 laurate, Polyglyceryl-5 laurate, Polyglyceryl-6 laurate, Polyglyceryl-7 laurate, Polyglyceryl-8 laurate, Polyglyceryl-9 laurate, Polyglyceryl-10 laurate. Bevorzugt sind die Verbindungen Polyglyceryl-4 caprate und/oder Polyglyceryl-6 caprate. Diese Verbindungen können beispielsweise unter der Handelsbezeichnung Tego Solve 90 MB bei der Firma Evonik Operations GmbH erworben werden.

Wenn in dem erfindungsgemäßen Haargel wenigstens ein nichtionisches Tensid enthalten ist, so liegt es vorteilhaft mit einem Gesamtgehalt von 0,1 bis 2,0 Gew.-%, bevorzugt 0,2 bis 1,0 Gew.-% vor, bezogen auf das Gesamtgewicht des Haargels und den Aktivgehalt.

In dem erfindungsgemäßen Haargel kann optional PEG-40 gehärtetes Rizinusöl (PEG 40 hydrogenated castor oil) enthalten sein. PEG-40 gehärtetes Rizinusöl hat bedingt durch seinen amphiphilen Aufbau mehrere Funktionen, zu einen kann es als Emulgator und Lösungsvermittler (Lösungsmittel) wirken und zum anderem auch als Emollient.

Wenn in der erfindungsgemäßen Zubereitung PEG-40 gehärtetes Rizinusöl enthalten ist, so liegt es mit einem Gesamtgehalt von 0,1 bis 2,0 Gew.-%, bevorzugt 0,2 bis 1,0 Gew.-% vor, bezogen auf das Gesamtgewicht der Zubereitung.

Erfindungsgemäß vorteilhaft ist ebenfalls die Verwendung einer oder mehrerer Substanzen der Vitamine der B Gruppe und/oder des Vitamin B-Komplex, die dem erfindungsgemäßen Haargel zusätzlich zugesetzt werden können. Substanzen der Vitamin B Gruppe und/oder des Vitamin B-Komplex sind üblicherweise wasserlöslich und spielen insbesondere für den Zellmetabolismus bei Pflanzen und Tieren eine besondere Rolle.

Beispiele erfindungsgemäßer Substanzen der Vitamine der B Gruppe und/oder des Vitamin B-Komplex sind unter anderem Nicotinsäure (Vitamin B3), Nicotinsäureamid (Niacinamid), Panthenol (Provitamin B5); die Vitamine der B Gruppe und/oder des Vitamin B-Komplex sind vorteilhaft mit einem Gesamtgehalt von 0,01 bis 0,5 Gew.-% enthalten, bezogen auf das Gesamtgewicht des Haargels.

Das erfindungsgemäße Haargel kann vorteilhaft Konservierungsmittel enthalten. Konservierungsmittel sind diejenigen konservierenden Substanzen, die gemäß Kosmetikverordnung für Deutschland und gemäß der Verordnung (EG) Nr. 1223/2009 über kosmetische Mittel für den Einsatz in kosmetischen Produkten für Europa zugelassen sind.

Das wenigstens eine Konservierungsmittel kann vorteilhaft ausgewählt werden aus der Gruppe Phenoxyethanol, Methylparaben, Polyaminopropyl Biguanide, Benzoesäure und/oder ein physiologisch verträgliches Salz davon, Benzethonium Chlorid und/oder Benzyl Alcohol, bevorzugt Benzoesäure und/oder ein physiologisch verträgliches Salz davon, weiter bevorzugt Natriumbenzoat.

In dem erfindungsgemäßen Haargel liegt das wenigstens eine Konservierungsmittel mit einem Gesamtgehalt von 0,0001 bis 2 Gew.-%, bevorzugt von 0,0005 bis 1,5 Gew.-%, besonders bevorzugt von 0,001 bis 1 Gew.-% vor, bezogen auf das Gesamtgewicht des Haargels.

Weiterhin kann das erfindungsgemäße Haargel wenigstens einen Stabilisator enthalten. Im Sinne der vorliegenden Erfindung sind als Stabilisatoren diejenigen Substanzen zu verstehen, die nicht in der Liste der zugelassenen Konservierungsstoffe (Kosmetikverordnung Anlage 6; Verordnung (EG) Nr. 1223/2009, Anhang V) aufgeführt sind, gleichwohl aber eine stabilisierende Wirkung für die entsprechende Zubereitung haben und/oder im gemeinsamen Einsatz mit Konservierungsmitteln die Stabilität der Zubereitung fördern.

Als Stabilisatoren sind folgende Verbindungen vorteilhaft: Propylenglycol, Ethylhexylglycerin, 1,2-Hexanediol, Methylpropanediol, Butylene Glycol, Caprylyl Glycol, Glyceryl Caprylate, Hydroxyacetophenone und/oder Pentylene Glycol.

Das erfindungsgemäße Haargel kann vorteilhaft Parfüm enthalten. In der Regel ist das eingesetzte Parfüm eine Mischung aus Parfümrohstoffen. Das Parfüm ist hauptsächlich enthalten, um dem erfindungsgemäßen Haargel einen angenehmen Geruch zu verleihen.

Das erfindungsgemäße Haargel kann in jedem geeigneten und in der kosmetischen Industrie üblichen Verpackungsmittel bereitgestellt werden. So ist eine Ausgestaltungsform des erfindungsgemäßen Haargel derart, dass das Haargel in einem Tiegel angeboten wird. Der Tiegel kann aus den üblichen Materialien bestehen, wie sie in der kosmetischen Industrie gebräuchlich sind, beispielsweise aus Kunststoff (PET, PP oder PS), Glas oder Metall, beispielsweise Aluminium.

Vorteilhaft wird das Haargel jedoch so ausgestaltet, dass eine Entnahme aus einer Tube erfolgen kann. Die Tube kann aus den üblichen Materialien bestehen, wie sie in der kosmetischen Industrie gebräuchlich sind, beispielsweise aus Kunststoff (PP, C/LDPE oder LDPE, HDPE) oder Metall (Aluminium). Um eine Entnahme aus der Tube zu gewährleisten, ist die Viskosität des Haargels vorteilhaft im Bereich von 10.000 bis 30.000 mPa·s gewählt.

Die Bestimmung der Viskosität erfolgt bei 25°C in einer 150 ml Weithalsflasche (VWR Nr.: 807-001) mittels Rheomat R 123 von der Firma proRheo. Der Rheomat R 123 der Firma proRheo GmbH ist ein Rotationsviskosimeter, d. h. ein Messkörper rotiert in der zu vermessenden Substanz. Es wird die Kraft gemessen, die benötigt wird, um den Messkörper in der Probe mit einer vorgegebenen Drehzahl rotieren zu lassen. Aus diesem Drehmoment, der Drehzahl des Messkörpers und den geometrischen Abmessungen des verwendeten Messsystems wird die Viskosität berechnet. Als Messkörper wird der Messkörper Nr. 2 (Artikelnr. 200 0192), geeignet für einen Viskositätsbereich 7.500 bis 300.000 [mPa·s], Drehzahl Bereich 62,5 min-1, verwendet.

Um zu zeigen, dass die Merkmale des Gegenstandes der Erfindung, nämlich ein Haargel enthaltend Pullulan in einer bestimmten Menge, optional eine Film- bildende und/oder festigende Verbindung, einen natürlichen Verdicker und optional wenigstens ein nichtionisches Tensid und/oder PEG-40 Hydrogenated Castor Oil, einen Beitrag zur Lösung der Aufgaben leisten, wurden verschiedene Haargele (Tabelle 1, Beispiele 1 bis 5) hergestellt. Ein Haargel gemäß Beispiel 1 enthielt als Struktur-gebende Verbindung ein Acrylates/Hydroxyesters Acrylates Copolymer und als Film-bildende und verdickende Verbindung Acrylates/Steareth-20 Methacrylate Crosspolymer; dieses Haargel ist eine Vergleichszubereitung gemäß dem StdT. Ein Haargel gemäß Beispiel 2 enthält Pullulan und ein synthetisches Film-bildendes und verdickendes Polymer, die Haargele gemäß den Beispiele 3 bis 5 enthalten nur natürliche Komponenten. Die Haargele wurden auf 3D-Tressen aufgetragen und die Halteeigenschaft untersucht.

3D-Haartressen sind aus Euro-Natur-Haar aufgebaut, die auf ein Silikonblatt (4 × 24,5 cm) gestochen werden, wobei davon 3 × 10 cm bestochen und etwa 3 Haare pro Loch enthalten sind. Die Haarlänge beträgt etwa 3 cm und das Haargewicht etwa 1,2 bis 1,4 g.

Es zeigte sich, dass eine Kombination von Pullulan mit einem natürlichen Verdicker, ausgewählt aus der Gruppe Xanthan Gum, Algin und/oder Carrageenan, wenigstens einer Film-bildende und/oder festigende Verbindung, insbesondere Natrium Polyitaconate, und wenigstens einem nichtionischen Tensid überaus befriedigende Ergebnisse lieferte (Beispiel 5). Die Verteilung des Haargels auf dem Haar und die fixierenden Eigenschaften wurden als sehr gut beurteilt. Die Halteeigenschaften des o.g. Haargels wurden am besten bewertet.

Die behandeltet 3D-Tresse wurde nach dem Trocknen mit der Handinnenfläche mit etwas Druck belastet. Je mehr Widerstand spürbar ist, desto stärker ist der Halt des Haargels. Drei erfahrenen Labormitarbeiter*innen führten die Bewertung durch. Die Bewertung erfolgte in Stufen, hierbei wurde den Vergleichszubereitungen 1 und 2 der Wert 0 zugeordnet. Eine bessere bzw. schlechtere Bewertung als diejenige der Vergleichszubereitung wurde folgendermaßen abgestuft:

| | |
|---|---|
| deutlich schlechter | --- |
| schlechter | -- |
| etwas schlechter | - |
| Standard | 0 |
| etwas besser | + |
| besser | ++ |
| Deutlich besser | +++ |

Es wurde also gezeigt, dass ein Haargel, das nur natürliche und somit gut biologisch abbaubare Inhaltsstoffe enthält, mit einem Haargel des StdT vergleichbar ist, im Punkt Halteeigenschaft sogar deutlich besser bewertet wird.

Gegenstand der vorliegenden Erfindung ist also auch die Verwendung von Pullulan in einer Menge von 0,1 bis 15,0 Gew.-%, bezogen auf das Gesamtgewicht des Haargels, zusammen mit wenigstens einem natürlichen Verdicker, mit der Maßgabe, dass der natürliche Verdicker nicht Pullulan ist, in einem Haargel zur Festigung der Haare und zum Frisurerhalt.

Ebenso Gegenstand der vorliegenden Erfindung ist Verwendung von Pullulan in einer Menge von 0,1 bis 15,0 Gew. %, bezogen auf das Gesamtgewicht des Haargels zusammen mit wenigstens einem natürlichen Verdicker, ausgewählt aus der Gruppe Xanthan Gum, Algin und/oder Carrageenan und einem weiteren Film- bildenden und/oder festigenden Polymer, insbesondere Natrium Polyitaconate in einem Haargel zur Festigung der Haare und zum Frisurerhalt.

Bevorzugt ist es, wenn Natrium Polyitaconate mit einem Gehalt von 0,5 bis 15,0 Gew.-%, bezogen auf das Gesamtgewicht des Haargels und den Aktivgehalt enthalten ist.

### Beispiele:

Die nachfolgenden Beispiele sollen die vorliegende Erfindung verdeutlichen, ohne sie einzuschränken. Die Mengenangaben sind in Gew.-% und beziehen sich auf den Aktivgehalt, sofern nicht anders angegeben.

**Tabelle 1:**

| **INCI (Aktivgehalt)** | **1** | **2** | **3 4** | | **5** |
|---|---|---|---|---|---|
| Aqua | Ad 100 | Ad 100 | Ad 100 | Ad 100 | Ad 100 |
| Acrylates/Hydroxyesters Acrylates Copolymer | 1,75 | | | | |
| Pullulan | | 1,75 | 5 | 5 | 3 |
| Sodium Polyitaconate | | | | | 5 |
| Acrylates/Steareth-20 Methacrylate Crosspolymer | 1,45 | 1,45 | | | |
| Aminomethyl Propanol | 0,75 | 0,55 | | | |
| Xanthan Gum | | | 3 | 3 | 3 |
| Carrageenan | | | | 1,5 | 1,5 |
| Algin | | 3 | 3 | | |
| Sodium Benzoate | | | 1 | 1 | 1 |
| Panthenol | 1 | 1 | | | |
| PEG-40 Hydrogenated Castor Oil | 0,5 | 0,5 | | | |
| Caprylyl/Capryl Glucoside | | 0,5 | 0,5 | 0,5 | |
| Decyl Glucoside | | | | | 0,5 |
| Parfum | 0,3 | 0,3 | 0,3 | 0,3 | 0,3 |
| | | | | | |
| Halteeigenschaften | ο | ο | ++ | ++ | +++ |

## Patentansprüche

1. Haargel enthaltend
• Pullulan in einer Menge von 0,1 bis 15,0 Gew.-%,
• wenigstens einen natürlichen Verdicker und/oder wenigstens einen Verdicker natürlichen Ursprungs, mit der Maßgabe, dass der natürliche Verdicker nicht Pullulan ist,
• optional wenigstens ein weiteres Film- bildendes und/oder festigendes Polymer, wobei das Polymer ein natürliches Polymer oder ein Polymer natürlichen Ursprungs ist, mit der Maßgabe, dass das wenigstens eine weitere Film- bildende und/oder festigende Polymer nicht Pullulan, nicht der wenigstens eine natürliche Verdicker und/oder nicht der wenigstens eine Verdicker natürlichen Ursprungs ist und
• optional wenigstens ein nichtionisches Tensid und/oder PEG-40 gehärtetes Rizinusöl.

2. Haargel nach Anspruch 1, **dadurch gekennzeichnet, dass** der Wassergehalt von 70 bis 98 Gew.-%, bevorzugt von 75 bis 95 Gew.-% beträgt, bezogen auf das Gesamtgewicht des Haargels.

3. Haargel nach Anspruch 1 und/oder 2, **dadurch gekennzeichnet, dass** Pullulan mit einem Gehalt von 0,3 bis 10,0 Gew.-%, bevorzugt von 0,5 bis 7,0 Gew.-% enthalten ist, bezogen auf das Gesamtgewicht des Haargels.

4. Haargel nach wenigstens einem der vorstehenden Ansprüche, **dadurch gekennzeichnet, dass** der wenigstens eine natürliche Verdicker und/oder der wenigstens eine Verdicker natürlichen Ursprungs ausgewählt wird aus der Gruppe Agar-Agar, Carrageen, Tragant, Gummi arabicum, Alginsäure und/oder Salze der Alginsäure, Pektine, Guar-Mehl, Johannisbrotbaumkernmehl, Dextrine, Gelatine, Casein, Xanthan Gum, Gellan Gum, Succinoglycan Gum, Skleroglucan, bevorzugt aus der Gruppe Xanthan Gum, Carrageenan, Alginsäure und/oder Salze der Alginsäure.

5. Haargel nach wenigstens einem der vorstehenden Ansprüche, **dadurch gekennzeichnet, dass** der wenigstens eine natürliche Verdicker mit einem Gesamtgehalt von 0,1 bis 8,0 Gew.-%, bevorzugt 1,0 bis 6,0 Gew.-% vorliegt, bezogen auf das Gesamtgewicht des Haargels.

6. Haargel nach wenigstens einem der vorstehenden Ansprüche, **dadurch gekennzeichnet, dass** die wenigstens eine weitere Film- bildende und/oder festigende Verbindung Natrium Polyitaconat (INCI-Bezeichnung: Sodium Polyitaconate) ist.

7. Haargel nach Anspruch 6, **dadurch gekennzeichnet, dass** Natrium Polyitaconat mit einem Gehalt von 0,5 bis 15,0 Gew.-%, bevorzugt von 1,0 bis 8,0 Gew.-% enthalten ist, bezogen auf das Gesamtgewicht des Haargels.

8. Haargel nach wenigstens einem der vorstehenden Ansprüche, **dadurch gekennzeichnet, dass** die wenigstens eine Film- bildende und/oder festigende Verbindung das Polymer Acrylates/Steareth-20 Methacrylate Crosspolymer ist.

9. Haargel nach wenigstens einem der vorstehenden Ansprüche, **dadurch gekennzeichnet, dass** das nichtionische Tensid ausgewählt wird aus Alkylpolyglucosiden, insbesondere aus der Gruppe Laurylglucosid, Decylglucosid, Cocoglucosid und/oder Caprylyl/Capryl Glucoside.

10. Haargel nach wenigstens einem der vorstehenden Ansprüche, **dadurch gekennzeichnet, dass** das wenigstens eine nichtionisches Tensid mit einem Gesamtgehalt von 0,1 bis 2,0 Gew.-%, bevorzugt 0,2 bis 1,0 Gew.-% vorliegt, bezogen auf das Gesamtgewicht des Haargels und den Aktivgehalt.

11. Haargel nach wenigstens einem der vorstehenden Ansprüche, **dadurch gekennzeichnet, dass** PEG-40 gehärtetes Rizinusöl (PEG 40 hydrogenated castor oil) mit einem Gesamtgehalt von 0,1 bis 2,0 Gew.-%, bevorzugt 0,2 bis 1,0 Gew.-% vorliegt, bezogen auf das Gesamtgewicht der Zubereitung.

12. Haargel nach wenigstens einem der vorstehenden Ansprüche, **dadurch gekennzeichnet, dass** das Haargel keine Cellulose-Ether-Verbindungen enthält.

13. Haargel nach wenigstens einem der vorstehenden Ansprüche, **dadurch gekennzeichnet, dass** das Haargel keine Acrylat-basierten Polymere enthält.

14. Haargel nach wenigstens einem der Ansprüche 1 bis 13, **dadurch gekennzeichnet, dass** das Haargel
• Pullulan in einer Menge von 0,1 bis 15 Gew.-%, bezogen auf das Gesamtgewicht des Haargels,
• wenigstens einen natürlichen Verdicker und/oder wenigstens einen Verdicker natürlichen Ursprungs, ausgewählt aus Alginsäure und/oder ein oder mehrere Salze der Alginsäure und
• wenigstens ein nichtionisches Tensid und/oder PEG-40 gehärtetes Rizinusöl, insbesondere ein nichtionisches Tensid,
umfasst.

15. Haargel nach wenigstens einem der Ansprüche 1 bis 13, **dadurch gekennzeichnet, dass** das Haargel
• Pullulan in einer Menge von 0,1 bis 15 Gew.-%, bezogen auf das Gesamtgewicht des Haargels,
• wenigstens einen natürlichen Verdicker und/oder wenigstens einen Verdicker natürlichen Ursprungs, ausgewählt aus der Kombination von Xanthan Gum und Carrageenan und
• wenigstens ein weiteres Film- bildendes und/oder festigendes Polymer, wobei das Polymer ein natürliches Polymer oder ein Polymer natürlichen Ursprungs ist, mit der Maßgabe, dass das wenigstens eine weitere Film- bildende und/oder festigende Polymer nicht Pullulan, nicht der wenigstens eine natürliche Verdicker und/oder nicht der wenigstens eine Verdicker natürlichen Ursprungs ist, insbesondere Natrium Polyitaconat,
umfasst.

16. Haargel nach wenigstens einem der Ansprüche 1 bis 13, **dadurch gekennzeichnet, dass** das Haargel
• Pullulan in einer Menge von 0,1 bis 15 Gew.-%, bezogen auf das Gesamtgewicht des Haargels,
• wenigstens einen natürlichen Verdicker und/oder wenigstens einen Verdicker natürlichen Ursprungs, ausgewählt aus der Kombination von Xanthan Gum und Carrageenan,
• wenigstens ein weiteres Film- bildendes und/oder festigendes Polymer, wobei das weitere Film- bildende und/oder festigende Polymer Natrium Polyitaconate ist,
• und wenigstens ein nichtionisches Tensid und/oder PEG-40 gehärtetes Rizinusöl, insbesondere ein nichtionisches Tensid,
umfasst.

17. Verwendung von Pullulan in einer Menge von 0,1 bis 15 Gew.-%, bezogen auf das Gesamtgewicht des Haargels, und wenigstens einem natürlichen Verdicker und/oder wenigstens einem Verdicker natürlichen Ursprungs, mit der Maßgabe, dass der natürliche Verdicker nicht Pullulan ist, in einem Haargel zur Festigung der Haare und zum Frisurerhalt.

18. Verwendung von Pullulan in einer Menge von 0,1 bis 15,0 Gew.-%, bezogen auf das Gesamtgewicht des Haargels, wenigstens einem natürlichen Verdicker und/oder wenigstens einem Verdicker natürlichen Ursprungs, mit der Maßgabe, dass der natürliche Verdicker nicht Pullulan ist, und wenigstens einem weiteren Film- bildenden und/oder festigenden Polymer, insbesondere Natrium Polyitaconate in einem Haargel zur Festigung der Haare und zum Frisurerhalt.

19. Verwendung nach Anspruch 19, **dadurch gekennzeichnet, dass** Natrium Polyitaconate mit einem Gehalt von 0,5 bis 15,0 Gew.-%, bezogen auf das Gesamtgewicht des Haargels und den Aktivgehalt, enthalten ist.
